# EUROPÄISCHE PATENTSCHRIFT

(11) **EP 1 318 751 B1**
(45) Veröffentlichungstag und Bekanntmachung des Hinweises auf die Patenterteilung: **23.03.2005**
(21) Anmeldenummer: 01984624.5
(22) Anmeldetag: 17.09.2001
(51) Int. Cl.: A61B 5/113, A61B 5/08

(54) **VORRICHTUNG UND VERFAHREN ZUR ERZEUGUNG VON ATMUNGSBEDINGTEN MESSDATEN**
DEVICE AND METHOD FOR PRODUCING RESPIRATION-RELATED DATA
PROCEDE ET DISPOSITIF DE PRODUCTION DE DONNEES DE MESURE RELATIVES A LA RESPIRATION

(30) Priorität: 15.09.2000 DE 10046075
(43) Veröffentlichungstag der Anmeldung: 18.06.2003
(73) Patentinhaber: Friendly Sensors AG, 07743 Jena (DE)
(72) Erfinder: FRIEDRICHS, Arnd, 07743 Jena (DE); VOEGELI, Fridolin, CH-8800 Thalwil (CH)
(74) Vertreter: Behrmann, Niels, Dipl.-Ing.
(86) Internationale Anmeldenummer: PCT/EP2001/010696
(87) Internationale Veröffentlichungsnummer: WO 2002/022017

(56) Entgegenhaltungen:
- US-A- 4 576 179
- US-A- 4 966 155
- US-A- 4 967 751
- US-A- 5 588 439

## Beschreibung

Die vorliegende Erfindung betrifft eine Vorrichtung sowie ein Verfahren zum Erzeugen von Messdaten betreffend atmungsbedingte Bewegungen der Bauchdecke und/oder des Brustkorbes einer Person. Dabei betrifft die vorliegende Erfindung insbesondere eine technische Lehre, welche die gezielte Erfassung und Überwachung der Atmungstätigkeit eines Menschen zum Gegenstand hat.

Bekanntermaßen erfolgt die Einatmung (Inspiration) durch Erweiterung des Brustkorb-Lungenraumes durch die Atemmuskeln sowie durch die resultierende Entwicklung eines relativen alveolären Unterdrucks in der sich ausdehnenden Lunge, wodurch es zu einem Lufteinstrom bis zum Druckausgleich kommt. Die Ausatmung (Exspiration) erfolgt überwiegend durch passive Verkleinerung des Thoraxraumes, und zwar durch Brustkorbsenkung sowie durch elastizitätsbedingte Volumenabnahme (Retraktion) der Lunge, wodurch Luft in Folge eines entstehenden, relativen alveolären Überdrucks ausströmt. Typische Störungen dieses menschlichen Atmungsvorganges ergeben sich etwa durch Elastizitätsabnahme der Lunge oder durch Einengung der Bronchiallichtungen (sog. restriktive bzw. obstruktive Ventilationsstörungen), sowie durch mögliche Störungen des Atemzentrums, der Diffusion oder der Blutzirkulation in der Lunge.

Zwerchfellatmung ist der durch Zwerchfellkontraktion entstehende Anteil an der Atmung (etwa zwei Drittel des Atemvolumens).

Aus dem Stand der Technik sind Ansätze bekannt, um eine atmungsbedingte Brust- und Bauchbereichsausdehnung bei der menschlichen Atmung zu erfassen und zu messen. Typischerweise hier eingesetzte Sensoren arbeiten auf piezoelektrischen Prinzipien und erzeugen eine vergleichsweise niedrige Spannung als Ausgangssignal, wobei bei einer Atembewegung auf einen solchen Sensor durch die atmungsbedingte Erweiterung der Brust- oder Bauchpartie eine Kraft ausgeübt wird. Aus dem dadurch erzeugten Spannungssignal wird durch geeignete Aufbereitung und Weiterverarbeitung ein Atmungssignal generiert.

Andere, aus dem Stand der Technik bekannte Systeme arbeiten auf einem Impedanzprinzip, d. h. unter Einsatz geeigneter Widerstandselemente, deren elektrischer Widerstand sich durch (atmungsbedingte) Bewegungen des Brustkorbes oder des Bauchbereichs ändert; insbesondere finden hier Dehnungsmessfühler oder dergl. Sensoren Einsatz.

Allerdings weisen derartige, bekannte Ansätze den Nachteil auf, dass nur eine summarische Erfassung der menschlichen Atemsignale möglich ist, wobei Qualität und Auflösung des erhaltenen elektronischen Signals im Normalfall nicht ausreichen, um mit vertretbarem Aufwand weitere Körperparameter -- ohne gesonderte, zusätzliche Sensoren -- überwachen zu können.

Insbesondere aufgrund eines inhärenten biologischen Zusammenhanges etwa zwischen der Atmung und der Herztätigkeit wäre es daher wünschenswert, mit geringem Aufwand, insbesondere unter Einsatz lediglich eines Sensors bzw. einer Sensoranordnung, beide Parameter gleichzeitig überwachen zu können. Entsprechendes gilt für Bewegung oder Aktivität der Person, wie es insbesondere auf dem Gebiet der Beobachtung von sportlichen Aktivitäten wünschenswert sein könnte.

Aus der US-A-5 588 439 ist eine Vorrichtung nach dem Oberbegriff des Hauptanspruchs bekannt; eine derartige Vorrichtung dient der Erzeugung von Messdaten betreffend atmungsbedingter Bewegungen der Bauchdecke und des Brustkorbs einer Person, wobei mindestens ein Paar von Sensoreinheiten und eine damit verbundene Messeinrichtung zum Erfassen elektronisch auswertbarer Signale der Sensoreinheiten vorgesehen ist. Es wird ein Abstandssignal erzeugt und durch eine Auswerteeinheit ausgewertet, wobei periodisch Signaländerungen als Atmungsanzeigesignal ausgegeben werden können.

Aufgabe der vorliegenden Erfindung ist es daher, eine Vorrichtung zur Erzeugung von Messdaten betreffend atmungsbedingte Bewegungen der Bauchdecke und/oder des Brustkorbes einer Person zu schaffen, die im Hinblick auf die Signalauflösung und Messgenauigkeit bei der Erzeugung eines Atmungsanzeigesignals verbessert ist, damit insbesondere auch Möglichkeiten bietet, aus diesem Atmungsanzeigesignal weitere Parameter bzw. überlagerte Signale zu detektieren und die Möglichkeit zu schaffen, neben auf die Atmungsbewegung zurückgehenden Messdaten weitere Messdaten mit derselben Sensorik zu generieren, die anderen Körperparameter bzw. -funktionen, eingeschlossen dem Herzschlag, entsprechen.

Die Aufgabe wird durch die Vorrichtung mit den Merkmalen des Patentanspruchs 1 sowie das Verfahren mit den Schritten des Patentanspruchs 11 gelöst. Vorteilhafte Weiterbildungen der Erfindung sind in den Unteransprüchen beschrieben.

In erfindungsgemäß vorteilhafter Weise macht sich die vorliegende Erfindung zur Messdatenerfassung betreffend atmungsbedingte Bewegungen der Bauchdecke und/oder des Brustkorbes einer Person das Prinzip einer Messdatenerfassung durch Entfernungsmessung zwischen einem Paar von Sensoreinheiten zunutze, wobei Änderungen im Abstand der Sensoren, wie sie durch die zu detektierende, atmungsbedingte Bewegung induziert werden, das entsprechend auswertbare erfindungsgemäße erste Abstandssignal erzeugen. In diesem Zusammenhang ist die Befestigung der Sensoren auf der Haut als so über der Haut bzw. Körperoberfläche zu verstehen, dass deren Abstandsänderung bezogen auf ein Meßpunktepaar erfassbar ist; es ist daher auch insbesondere vorgesehen, erfindungsgemäß die Sensoren über eine Zwischenlage -- Kleidungsstück , Tuch od. dgl. -- auf der Haut zu befestigen.

Dieses Prinzip der Erzeugung des ersten Abstandssignals entspricht dem in der deutschen Patentanmeldung 42 14 523 beschriebenen und ist mittels eines auf Ultraschall bzw. elektromagnetischen Wellen basierenden Sender-Empfänger-System in ansonsten bekannter Weise realisiert.

Für eine Realisierung der erfindungsgemäßen Messdatenerzeugung betreffend atmungsbedingte Bewegungen hat sich dabei das Prinzip der Ultraschall-Abstandsmessung bewährt, bei welchem die durch Bewegungen der Bauchdecke bewirkten Laufzeitunterschiede im Ultraschallsignal zwischen den Sensoreinheiten gemessen und ausgewerten werden; alternativ ist es möglich, Phasendifferenzen zwischen gesendetem und empfangenem Signal zu verwenden und auszuwerten.

Gemäß der vorliegenden Erfindung findet nunmehr diese bekannte Technologie Anwendung auf die besonderen Anforderungen der Messung einer atmungsbedingten Bauchdecken- bzw. Brustkorbbewegung, wobei, zusammen mit der erfindungsgemäß vorgesehenen Auswerteeinheit, gerade die abstandsabhängige Messdatenerfassung von Bewegungen des Brustkorbes sich als besonders zuverlässig und präzise herausgestellt hat. Vorteilhaft erlaubt nämlich dieses erfindungsgemäße Vorgehen nicht nur das (generelle) Überprüfen auf das Vorliegen einer Atembewegung (welches erfindungsgemäß durch die mit der Auswerteeinheit ermöglichte signalbezogene bzw. zeitbezogene Diskriminierung erfolgt), auch bietet die Erfindung, insbesondere, wenn weiterbildungsgemäß eine Mehrzahl von Sensorpaaren vorgesehen werden, die durch Atembewegungen induzierten Brustkorbbewegungen mit deren zeitlichen, örtlichen und räumlichen Ausbreitungen mit hoher Auflösung zu erfassen und so eine Untersuchung dahingehend zu ermöglichen, dass ein Atemvorgang selbst möglicherweise pathologisch sein könnte (indem etwa eine detektierte, hochaufgelöste Signalform des Atmungssignals nicht einer Norm entspricht).

Im Rahmen der Erfindung hat sich zudem herausgestellt, dass die Signalform einer normalen Atmung im Signal-Zeitdiagramm symmetrisch ist, also eine ansteigende sowie eine abfallende Flanke eines durch Abstandsänderung gemäß der Erfindung gemessene Atemsignals bezogen auf einen Mittelwert symmetrisch liegen. Vorgeschen ist es daher, der Auswerteeinheit Signalformerfassungsmittel zuzuordnen, die, zur Abgrenzung von anderen Signalen (etwa durch Sensorbewegung erzeugte Artefakte, Körperlagesignale oder Herzbewegungen) mit hoher Genauigkeit und Störunempfindlichkeit das Vorliegen von Atemtätigkeit erkennen und die Anzeigegenauigkeit weiter verbessern.

Gemäß einer weiteren, bevorzugten Weiterbildung (best mode) sind mindestens zwei Paare von Sensoreinheiten vorgesehen, die, weiter bevorzugt, jeweils eine sich kreuzende Strecke aufspannen (z. B. einen in etwa rechten Winkel ausbilden) und den Brustkorb oder Bauchraum durchstrahlen. Besonders günstig lassen sich so die atmungsinduzierten, lokal (und ggf. temporär) unterschiedlichen Brustkorbbewegungen berücksichtigen und, z. B. durch Summen- bzw. Differenzbildung der aus beiden Sensorpaaren enthaltenen Signale, für eine noch genauere Messung auswerten.

Generell ist es zudem von der vorliegenden Erfindung umfasst, die Sensoreinheiten eines jeweiligen Paares von Sensoreinheiten entweder so zueinander auszurichten, dass eine Verbindungslinie außerhalb bzw. entlang einer Peripherie des Brustkorbes bzw. Bauchbereichs verläuft, oder die Verbindungslinie den Brustkorb bzw. Bauchbereich längs oder diagonal durchdringen zu lassen.

Gemäß einer bevorzugten Weiterbildung der Erfindung ist vorgesehen, neben dem erfindungsgemäß erfassten und ausgegebenen, atmungsinduzierten Signal zusätzlich ein Herzfrequenzsignal der zu überwachenden Person zu erfassen, wobei dies im Rahmen der Erfindung ebenfalls durch Auswertung des durch die erfindungsgemäße Abstandsmessung gewonnenen Abstandssignals geschieht: Wie sich vorteilhaft herausgestellt hat, führt auch Herztätigkeit (mit einer Herzfrequenz im Bereich von typischerweise ca. 1 Hz) zu periodischen Bewegungen des Brustkorbes, wobei jedoch diese Signale im Rahmen der Erfindung zuverlässig von den für Atmung charakterischen Abstandssignalen frequenz- und/oder amplitudenmäßig diskriminiert werden können. Eine zusätzliche aktive Überwachung der Herztätigkeit, in Verbindung mit einer Atmungsüberwachung, ermöglicht es daher nicht nur, einen etwa diagnostischen Wert des eigentlichen Atemsignals zu erhöhen, sondern gestattet auch die zuverlässige Detektion weiterer kritischer Zustände der Person, wie etwa Schlafapnoe oder respiratorische Sinusarrhythmie, mithin das rechtzeitige Einleiten etwaiger Gegenmaßnahmen. Vorteilhaft sind zusätzliche Sensoren zur Herzschlagdetektion unnötig, die, neben apparativem Aufwand und potentiellen Problemen bei der Fixierung am Patienten, zusätzliche Hürden bei einer gemeinsamen Signalauswertung mit einem atmungsabhängigen Signal bieten würde.

Gemäß einer weiteren bevorzugten Weiterbildung der Erfindung ist vorgesehen, das erste Abstandssignal im Hinblick auf erschütterungsbedingte Signaländerungen auszuwerten, wie sie durch Schritte, Laufen oder Hüpfen der Person erzeugt werden und, wie sich im Rahmen der vorliegenden Erfindung ergeben hat, auch zuverlässig detektierbar und von dem Atmungsanzeigesignal diskriminierbar sind (insbesondere als bei typischen Schrittfrequenzen im Bereich von ca. 2,5 Hz sich diese Signale bereits im Frequenzbereich zuverlässig von den für Atmung und Herztätigkeit charakteristischen Änderungen des ersten Abstandssignals unterscheiden lassen). Eine zusätzliche, durch diese Weiterbildung der Erfindung ermöglichte aktive Überwachung der Schrittfrequenz gestattet es daher, ergänzend auch Zustände einer physischen Aktivität der Person, wie Trainingszustand, Abhängigkeiten zwischen Herzfrequenz, Atemfrequenz und Schrittfrequenz zuverlässig zu erfassen, zu analysieren und die anschließenden diagnostischen Maßnahmen zuzuführen. Weisen Herzfrequenzsignal und Schrittfrequenzsignal die gleichen Bereiche auf, können diese durch unterschiedliche Amplituden diskriminiert werden.

Gemäß einer weiteren bevorzugten Weiterbildung der Erfindung hat sich herausgestellt, dass eine Hüllkurve des Atmungsanzeigesignals, und dort insbesondere die oberen und unteren Grenzwerte dieser Hüllkurve (die ja einem maximalen bzw. minimalen Abstand zwischen dem Paar von Sensoreinheiten entsprechen) sich charakteristisch ändern, wenn die Person, etwa in einer Schlafposition, ihre Lage ändert -- sich etwa von der Rücken- in die Seitenlage dreht. Einer entsprechenden Weiterbildung gemäß vorgesehene Auswertung dieser Hüllkurvenparameter über einen typischerweise mehrminütigen Zeitraum ermöglicht daher zusätzlich das Ableiten von Lage- bzw. Positionsdaten der Person aus dem Abstandssignal.

Besonders geeignet ist die vorliegende Erfindung zur Verwendung mit einer portablen, und weiter bevorzugt drahtlos datenmäßig mit einer Basisstation zusammenarbeitenden Einheit: Die vorliegende Erfindung ermöglicht es nämlich, die erfindungsgemäß zur Messdatenerfassung und -auswertung vorgesehenen, üblicherweise mittels geeignet programmierbaren Controllern realisierten Einheiten in einem tragbaren, batteriebetriebenen Gehäuse vorzusehen, welches von der überwachten Person kontinuierlich mitgeführt wird, und welche das permanente Überwachen auf Atemtätigkeit, physische Aktivität der Person und Herzfrequenz ermöglicht. Hier eingeschlossen ist das Ermitteln eines Atem- und Belastungsprofils (Schlaf, Stress) sowie das Beobachten der Atmung in Abhängigkeit von verschiedenen Parametern.

Es sollte deutlich werden, dass durch die vorliegende Erfindung ein Instrument bzw. ein Verfahren geschaffen wird, welches nicht nur geeignet ist, auf einfach auszuwertende und hochgradig zuverlässige Art kritische von normalen Atemzuständen schnell zu unterscheiden (und damit sowohl schnelle Hilfe leisten zu können, als auch eine überflüssige Medikamentendarreichung zu vermeiden), durch eine portable Realisierung der Erfindung ist zudem in der Überwachung der Atemtätigkeit ein überaus vorteilhaftes Instrument zur Flexibilisierung und zur Komforterhöhung geschaffen.

Wie zudem durch die vorliegende Erfindung erreicht wird, kann mit einfachen Mitteln eine kombinierte Analyse verschiedener Körperfunktionen, eingeschlossen Atmung, Herztätigkeit sowie physischer Aktivität, erzeugt werden und detaillierte Aussagen über einen Gesamtzustand einer überwachten Person geben.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Fig. 1:: eine schematische Ansicht der erfindungsgemäßen Vorrichtung zur Erzeugung von atmungsinduzierten Messdaten gem. einer ersten Ausführungsform der Erfindung als Blockschaltbild mit den wesentlichen Funktionselementen und deren Zusammenwirken;
- Fig. 2:: eine Prinzipdarstellung als Frontansicht auf den Körper einer Person mit angelegter Vorrichtung gem. Fig. 1;
- Fig. 3:: eine rückwärtige Ansicht entsprechend Fig. 2;
- Fig. 4 bis 9:: verschiedene Prinzipdarstellungen zur Befestigung eines oder zweier Paare von Sensoreinheiten und Festlegung des ersten Abstandes als schematische Schnittansichten durch einen menschlichen Oberkörper, paarweise in ausgeatmetem und eingeatmetem Zustand;
- Fig. 10:: ein Signalzeitdiagramm zur Verdeutlichung des ersten Abstandssignals und dessen Änderungen in einem Ruhestand;
- Fig. 11:: eine Darstellung entsprechend Fig. 10, jedoch beim Laufen der Person;
- Fig. 12:: das Atemanzeigesignal in größerer zeitlicher Auflösung zur Verdeutlichung überlagerter Signale (Person steht);
- Fig. 13:: eine Darstellung analog Fig. 12 bei hüpfender Person;
- Fig. 14:: eine Darstellung analog Fig. 10 bis 13, jedoch mit verschiedenen Atmungsvorgängen sowie einer Überkreuz-Sensoranordnung analog Fig. 8, Fig. 9;
- Fig. 15:: eine ausschnittsweise vergrößerte Darstellung des rechtsgelegenen Abschnitts im Signalverlauf der Fig. 14;
- Fig. 16:: ein Signalzeitdiagramm zum Verdeutlichen des Atmungsanzeigesignals bei sportlicher Betätigung (Skaten), Sensoranordnung gem. Fig. 8, Fig. 9;
- Fig. 17:: ein Signalzeitdiagramm über eine ca. 50minütige Messdatenaufzeichnung bei schlafender Person mit Wechsel der Schlaflage und
- Fig. 18:: eine vergrößerte Ausschnittsdarstellung aus der Ansicht gem. Fig. 17 mit der Darstellung des Atmungsanzeigesignals im Schlaf.

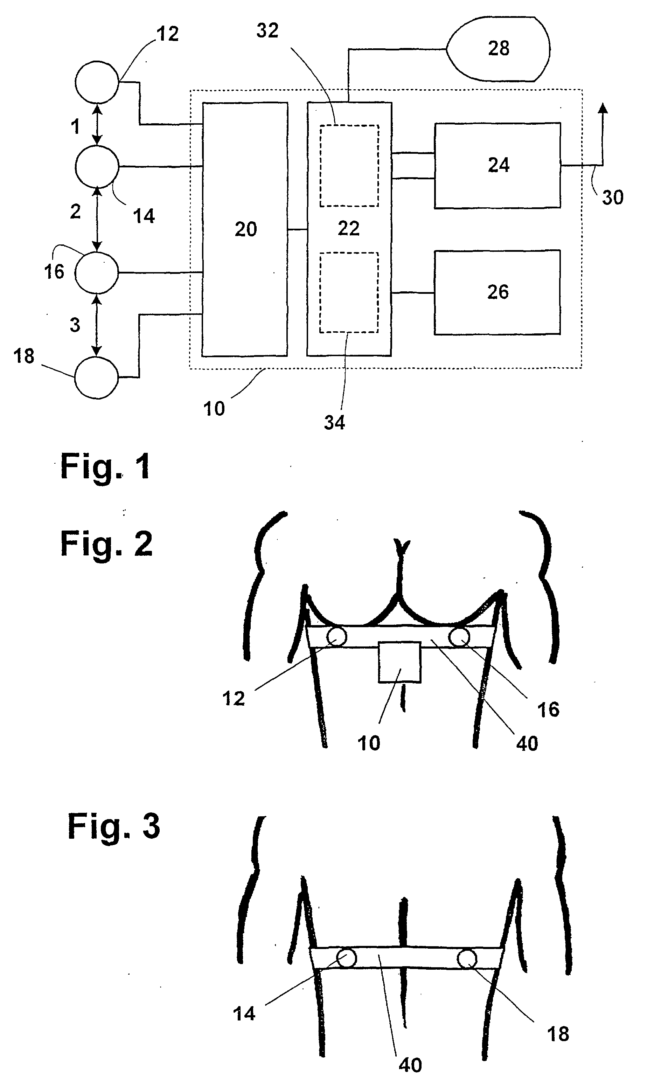

Figur 1 verdeutlicht in einer Blockschaltbilddarstellung, wie zwei Paare von Sensoreinheiten 12, 14 bzw. 16, 18 einer Ultraschall-Entfernungsmesseinheit 20 innerhalb eines portablen Gehäuses 10 zugeordnet sind, wobei, etwa gemäß Beschreibung der in Bezug genommenen Patentveröffentlichung DE 42 14 523, die Ultraschall-Entfernungsmesseinheit 20 in ansonsten bekannter Weise auf der Basis von Laufzeitunterschieden bei Abstandsänderungen eines Abstandes 1 (zwischen den Sensoreinheiten 12, 14) bzw. 3 (zwischen den Sensoreinheiten 16, 18; zusätzlich wird ein dritter Abstand 2 zwischen einer Sensoreinheit des ersten Paares und einer Sensoreinheit des zweiten Paares erfasst) ein entsprechendes, zur weiteren Auswertung geeignetes Signal ausgibt.

Konkret ist, wie in der Fig. 1 gezeigt, der Entfernungsmesseinheit 20 in schematischer Weise eine Auswerte- und Ausgabeeinheit 22 nachgeschaltet, die als Reaktion auf das (summarisch bzw. als Differenzsignal gebildete) Entfernungsmesssignal der Einheit 20 durch geeignete (bevorzugt rechnerisch am Signalbild bewirkte) Frequenzfilterung im Bereich von 0,1 bis 0,5 Hz das Atmungsanzeigesignal erzeugt und an eine Ausgabeeinheit 28, bevorzugt einen Monitor, ausgibt.

Parallel wird ein digitales Signalmuster des Atmungsanzeigesignals für spätere Auswertungen oder Korrelationen mit anderen Messwertverläufen in einer Speichereinheit 26 gespeichert, und über eine lediglich schematisch gezeigte Kommunikationseinheit 24 findet eine Anbindung des wie in Fig. 1 gezeigten, auf einfachstem Weg realisierten Atemmonitors an eine mittels Sendeantenne 30 drahtlos angebundene Basisstation zur weiteren Überwachung und Auswertung statt (etwa praktisch realisiert durch eine gängige GSM-Mobiltelefoneinheit).

Die Figuren 2 und 3 zeigen, wie die in Fig. 1 schematisch gezeigte Vorrichtung praktisch betrieben wird: Am schematisch gezeigten Körper eines Patienten ist im Brustkorbbereich ein Gurt 40 befestigt, mit welchem sowohl das Gehäuse 10, als auch die vier Sensoren 12 bis 18 so am Körper der Person befestigt werden können, dass diese zur gegenseitigen Abstandsmessung zusammenwirken können.

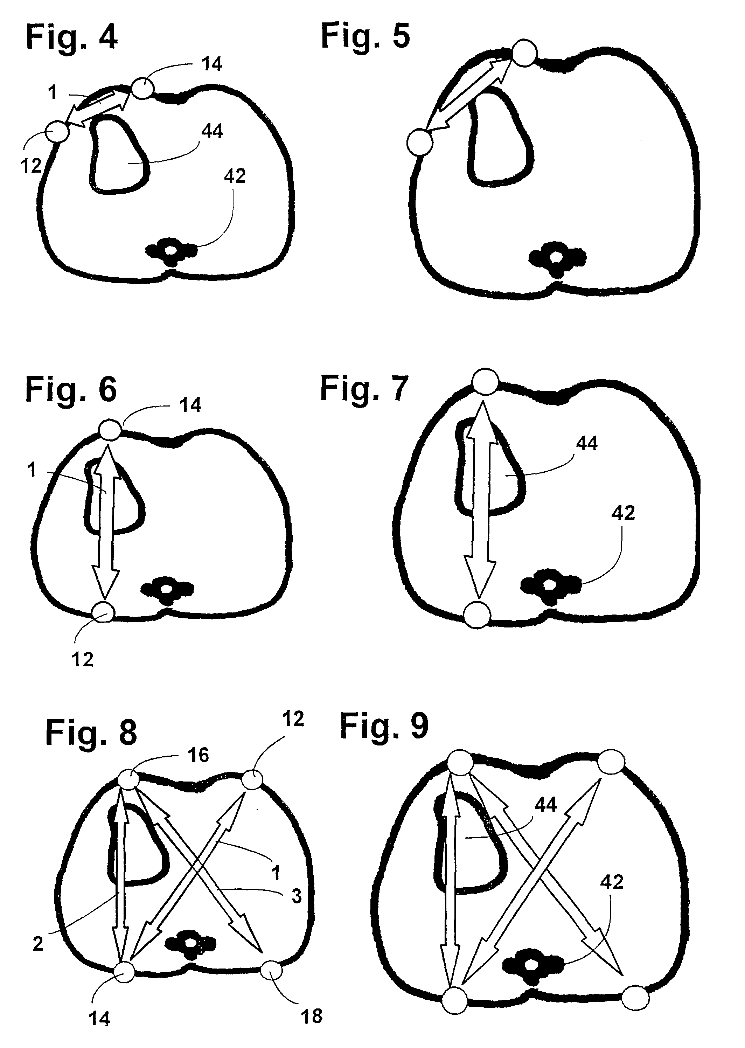

Unter Bezug auf die Figuren 4 bis 9 sollen im weiteren exemplarisch einige Möglichkeiten erörtert werden, Sensoreinheiten eines Paares sowie von zwei Paaren in der in Fig. 2, 3 skizzierten Weise am Körper zu befestigen, so dass gut auswertbare Signale erreichbar sind. Bei den Darstellungen der Figuren 4 bis 9 sind dabei die Sensoren eines bzw. zweier Paare von Sensoreinheiten jeweils als Kreise dargestellt, und die diese verbindenden Pfeile markieren die für eine Abstandsmessung bzw. Erzeugung von Abstandssignalen relevanten Strecken. Die Darstellungen sind horizontale Schnittdarstellungen durch den Brustbereich auf der Höhe des Gurtes 40 in Fig. 2, Fig. 3 wobei im Rückenbereich schematisch eine Wirbelsäule 42 und im vorderen linken Bereich das Herz 44 angedeutet ist.

Figur 4 verdeutlicht schematisch die Befestigung lediglich eines Paares von Sensoreinheiten gemeinsam an einem vorderen Brustbereich der Person, so dass die den Abstand (Pfeil 1) markierende Strecke randseitig am Körperäußeren verläuft. Während Fig. 4 den ausgeatmeten Zustand verdeutlicht, ist die Befestigung der Sensoreinheiten in Fig. 5 entsprechend, hier ist jedoch nach Einatmen der Brustkorb geweitet.

Die Figuren 6 und 7 (aus- bzw. eingeatmeter Zustand) verdeutlichen eine alternative Befestigungsmöglichkeit eines Sensorpaares: Hier wird ein erster Sensor des Paares im vorderen Brustbereich, der andere im Rückenbereich angeordnet, so dass eine den Abstand markierende Strecke (Pfeil 1) sich durch den Körper hindurch erstreckt. Durch die dadurch erreichte Vergrößerung des Sensorabstandes lässt sich gegenüber der Darstellung in Fig. 4, Fig. 5 unter Umständen eine weiter verbesserte Auflösung des atmungsinduzierten Abstandssignals erreichen.

Figur 8 zeigt eine Konfiguration mit zwei Paaren von Sensoreinheiten, die in der gezeigten Weise überkreuz im Brust- sowie Rückenbereich angeordnet sind; die Sensoranordnung der Schnittansichten gem. Fig. 8, Fig. 9 (wiederum aus- bzw. eingeatmeter Zustand) entspricht insoweit der in den Fig. 2 und 3 gezeigten Sensoranordnung sowie der Bezeichnung der Abstände 1, 2, 3 zwischen den einzelnen Sensoren gem. Fig. 1. Hier wird zudem ausgenutzt, dass zwischen den Sensoreinheiten 14 (des ersten Paares 12, 14) und 16 (des zweiten Paares 16, 18) eine zusätzliche Messstrecke 2 gebildet ist, was sich in der Praxis entweder durch Ultraschallsensoren mit mehreren Sensorelementen (Kristallen) realisieren lässt, durch mehrere an einer Stelle befestigte Sensoren, oder aber durch das Beschalten und Auswerten dergestalt, dass sich stets jeweils ein Sende- und ein Empfangselement gegenüberstehen (so könnte bei der Darstellung gem. Fig. 8, Fig. 9 etwa 12 ein Sende- und 14 ein Empfangselement sein, 16 wiederum ein Sende- und 18 ein Empfangselement, so dass auch zwischen 14 und 16 eine Sende-Empfangsstrecke zur Auswertung gebildet ist).

Durch geeignete Summen- bzw. Differenzbildung lässt sich insbesondere bei der in Fig. 8, Fig. 9 gezeigten Konfiguration ein optimierte Signalauflösung erreichen, die, wie nachfolgend anhand von Signalbildern zu besprechen sein wird, über das reine Vorliegen von Atmung hinaus in zahlreiche Parameter und Detailinformation auflös- und auswertbar ist.

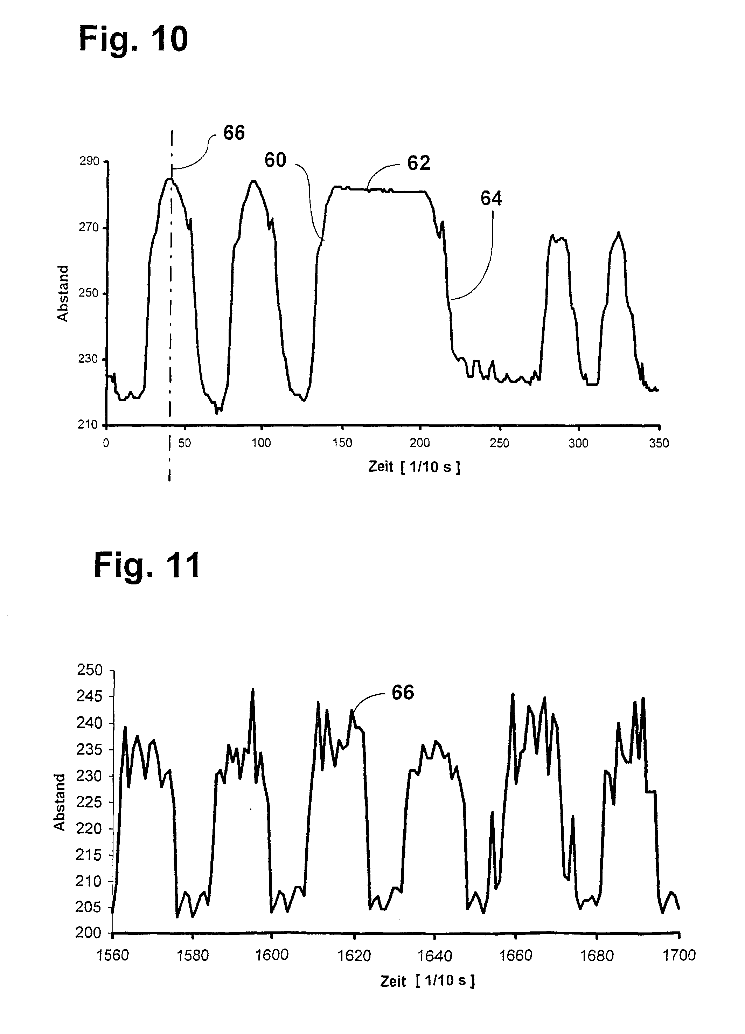

Die Darstellung in Fig. 10 zeigt insgesamt fünf Atemzyklen, wobei jeder Atmungszyklus durch eine steigende Flanke 60, einen maximalen Signalbereich 62 sowie eine fallende Flanke 64 gekennzeichnet ist, so dass, unabhängig von einer Atmungstiefe -die ersten beiden Atmungszyklen in der Abbildung gem. Fig. 10 stellen insoweit normale Zyklen dar, der mittlere Zyklus ist ein langer, besonders tiefer Atmungszyklus, und die beiden rechten Atmungszyklen sind sehr kurze, weniger tiefe Atmungszyklen -- eine symmetrische Signalform im Zeitbereich entsteht (exemplarisch ist eine Symmetrieachse 66 beim ersten Signal eingezeichnet).

Gegenüber der Darstellung in Fig. 10 weist das beim Laufen der Messperson ermittelte Diagramm der Fig. 11 charakteristische Überlagerungen des Atmungssignals mit einem Wechselsignal im Bereich der Lauffrequenz (ca. 2,5 Hz) auf; diese gegenüber dem (allerdings durch das Laufen beschleunigten) Atmungszyklus kürzeren Signale sind durch das Bezugszeichen 66 in Fig. 11 bezeichnet.

Wie die Fig. 11 zeigt, ermöglicht es die vorliegende Erfindung, nicht nur das Atmungssignal bei physischer Bewegung der Person (Laufen, Hüpfen usw) aufzuzeichnen; aufgrund der auftretenden Kraftstöße beim Bodenkontakt kommt es zusätzlich zu einer geringen Verschiebung der Sensorelemente im Lauf- bzw. Hüpfrhythmus, wobei diese Kraftspitzen in der Fig. 11 gut zu erkennen sind. Allerdings ist dieses überlagerte Signal sowohl frequenzals auch amplitudenmäßig von dem zugrunde liegenden Atmungssignal leicht zu trennen (um so eine getrennte Auswertung vornehmen zu können), und auch eine Übertragung dieses Auswertegedankens im Rahmen der Erfindung auf andere Wortarten (Radfahren, Inline-Skaten oder dergl.) erscheint möglich.

Im Rahmen des beschriebenen Ausführungsbeispiels gem. Fig. 1 erfolgt ein Auswerten dieser Schrittfrequenz mittels einer gesonderten Auswertungseinheit 34, die der Auswerte- und Ausgabeeinheit 28 zugeordnet ist.

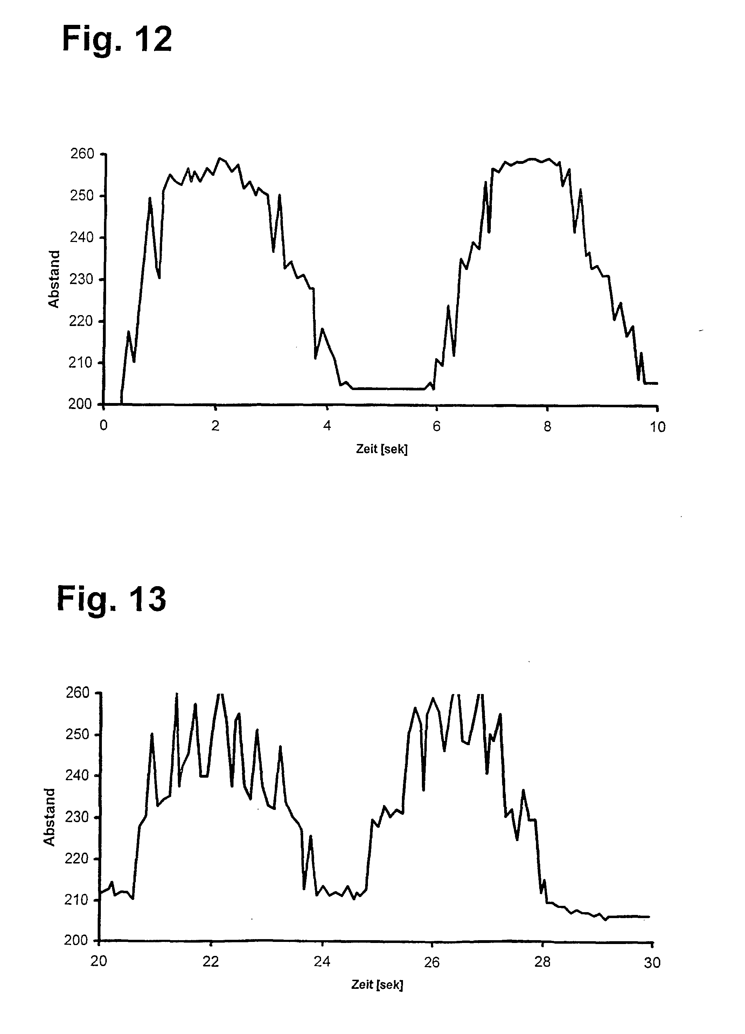

Figur 12 zeigt in größerer Auflösung im Signal-Zeitdiagramm das Atmungsanzeigesignal bei stehender Person (analog Fig. 10); Fig. 13 entspricht in der Auflösung der Fig. 11 und zeigt Atemanzeigesignale beim Hüpfen. Wiederum sind die durch die Kraftstöße induzierten Überlagerungen 66 gut zu erkennen.

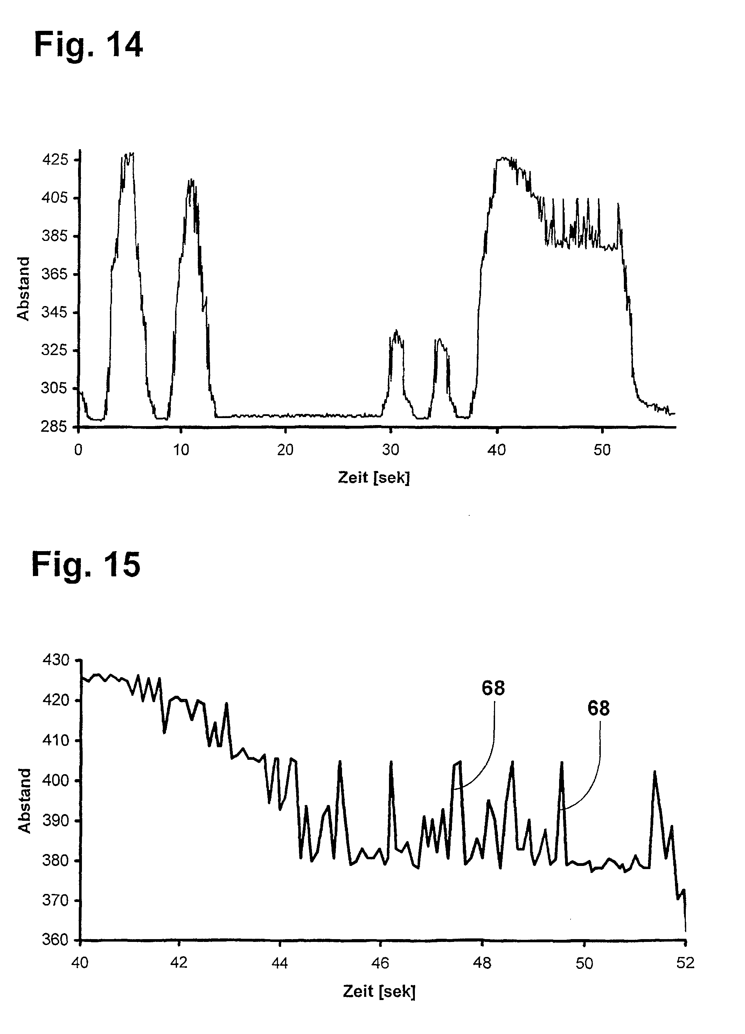

Die Figur 14 verdeutlicht ein Atmungsanzeigesignal mit einer Sensoranordnung (etwa Figur 6, Figur 7 oder Figur 8, Figur 9), bei welcher zur Verbesserung der Signalauflösung ein wirksamer Abstand zwischen einem Paar von Sensoreinheiten maximiert ist. Mit dieser verbesserten Auflösung lässt sich insbesondere Tiefe der Atmung erkennen und unterscheiden, so zeigen die in Fig. 14 links gelegenen Atemzyklen eine für eine tiefe Atmung charakteristische Wellenform, der sich anschließende, signalfreie Zeitabschnitt entspricht dem Anhalten des Atems nach dem Ausatmen, die sich anschließenden beiden Signale geringerer Höhe entsprechen einer normalen (weniger tiefen) Atmung, und der rechts gelegene, ausgedehnte und hohe Signalimpuls entspricht einem tiefen Einatmen und nachfolgendem Anhalten der Atmung.

Die Figur 15, die insoweit eine Ausschnittsvergrößerung des rechts gelegenen tiefen und angehaltenen Atemzyklus aus Figur 14 ist, verdeutlicht, wie überlagerte Herzschlagimpulse 68 in dem Signal erkennbar und durch geeignete Frequenz- und/oder Amplitudendiskriminierung zusätzlich erfass- und auswertbar sind. Zu diesem Zweck ist der Auswerte- und Ausgabeeinheit 22 in Fig. 1 eine separate Diskriminier- und Auswerteeinheit 32 zugeordnet, die in ansonsten bekannter Weise durch (bevorzugt numerische) Auswertung eines Signalverlaufs wie in Fig. 15 die Herzfrequenz ermittelt.

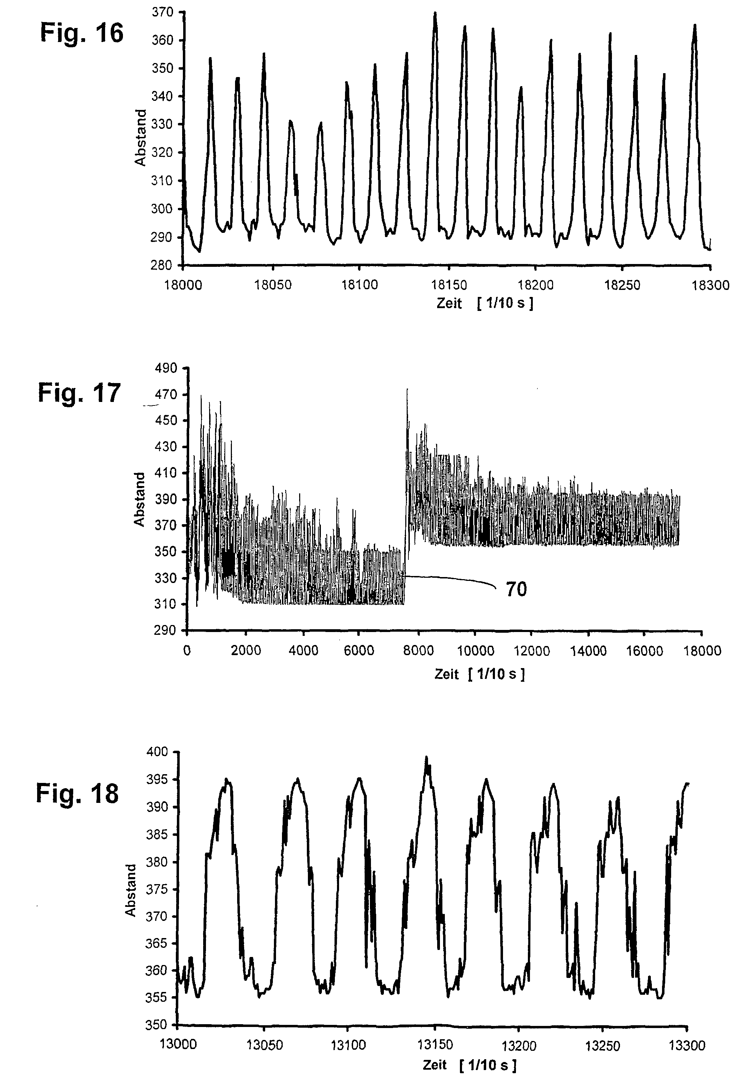

Figur 16 zeigt ein weiteres Beispiel für ein Atmungsanzeigesignal bei sportlicher Betätigung (hier: Skaten), die Figuren 17 (Langzeitmessung über ca. 50 Minuten) und Figur 18 (Auflösung aus Fig. 17 in einzelne Atmungszyklen) verdeutlichen Atmungssignale beim Schlafen einer Person.

Wie sich interessanterweise durch (bevorzugt numerische) Auswertung der Hüllkurve gem. Fig. 17 über einen mehrminütigen Zeitraum zeigt, wirken sich Lageänderungen des Schlafenden (z. B. ein Drehen von der Rücken- in die Seitenlage) in einem charakteristischen Sprung (Bezugszeichen 70 in Fig. 14) in der Hüllkurve des Atmungsanzeigesignals aus, genauer gesagt, dadurch, dass der Minimumabstand sprunghaft und dauerhaft sich ändert. Entsprechend lässt sich durch geeignete (bevorzugt numerische) Auswertung der Hüllkurve auch eine derartige Lageänderung eines Schlafenden aus dem ohnehin vorhandenen Abstandssignal ermitteln und in weitere Auswertungen als Grundlage für diagnostische Zwecke einfließen.

Es zeigt sich daher bereits aus der Betrachtung der Ausführungs- und Anwendungsbeispiele, dass die vorliegende Erfindung Potential für eine Vielzahl von Anwendungsmöglichkeiten bietet; diese reichen von einer Langzeit-Atemmessung sowie einer Langzeitaktivitätsmessung über die Berücksichtigung von Atem-, Puls- und Bewegungsdaten im Biofeedback sowie im Stressmanagement, über das kontrollierte Atemtraining bei Schwangeren, das Schrittzählen beim Joggen, die Überwachung von Atemstörungen verschiedenster Art (eingeschlossen Überwachung des SIDS, des plötzlichen Kindstods), bis hin zur Ermittlung von Kreislaüfparametern unter Belastung (eingeschlossen Energieumsatzmessung), wie etwa des Puls-Atemquotienten, und dies lediglich durch signalmäßige Auswertung eines einzigen, gemäß der vorliegenden Erfindung erhaltenen, gleichwohl hochaufgelösten und damit aussagekräftigen Entfernungs- bzw. Abstandssignals. Damit lassen sich als bestimmbare Messgrößen neben Atemfrequenz und Atemtiefe (hier besteht ein Zusammenhang mit dem Lungenvolumen) auch die Atemvariabilität ermitteln, durch geeignete Positionierung der Sensoreinheiten etwaige Unterschiede zwischen der Funktionsfähigkeit des rechten und des linken Lungenflügels, Unterschiede zwischen Tag- und Nachtaktivitäten im Hinblick auf Atmung, Puls usw., Lageänderungen beim Schlafen, die Schritt- und Herzfrequenz (um aus einer Kombination von Atem und Herzfrequenz auf den Energieumsatz der Person zu schließen) sowie ein Verhältnis zwischen Bauch- und Brustatmung.

## Patentansprüche

1. Vorrichtung zur Erzeugung von Messdaten betreffend atmungsbedingte Bewegungen der Bauchdecke und/oder des Brustkorbes einer Person, mit
mindestens einem ersten Paar von Sensoreinheiten (12, 14), die zum lösbaren Befestigen auf der Haut an einem Brustkorb- oder Bauchbereich der Person, beabstandet um einen ersten Abstand (1), ausgebildet sind,
und einer mit dem ersten Paar von Sensoreinheiten verbundenen Messeinrichtung (20), die zum Erfassen elektrisch auswertbarer Signale der Sensoreinheiten sowie zum Erzeugen eines dem ersten Abstand (1) sowie Änderungen desselben entsprechenden ersten Abstandssignals ausgebildet ist,
wobei der Messeinrichtung eine Auswerteeinheit (22, 28) nachgeschaltet ist, die zum Auswerten impulsund/oder wellenförmiger Änderungen des ersten Abstandssignals dergestalt ausgebildet ist, dass periodische Signaländerungen erfasst, von periodischen, durch den menschlichen Herzschlag bestimmten Änderungen des ersten Abstandssignals frequenz-, amplitudenoder signalformmäßig unterschieden und als elektronisch anzeig- oder weiter auswertbares Atmungsanzeigesignal ausgegeben werden können, **dadurch gekennzeichnet, dass** die Signaländerungen eine Frequenz im Bereich zwischen 0,5 und 0,1 Hz aufweisen und die Auswerteeinheit Mittel zur Erfassung einer Signalform der Änderung des ersten Abstandssignals aufweist, wobei die Signalformerfassungsmittel so ausgebildet sind, dass das Atmungsanzeigesignal nur als Reaktion auf eine im Zeitablauf symmetrische Signalform der Änderung ausgegeben wird.

2. Vorrichtung nach Anspruch 1, **gekennzeichnet durch** mindestens ein mit der Messeinrichtung verbundenes zweites Paar (16, 18) von Sensoreinheiten, die zum lösbaren Befestigen auf der Haut an dem Brustkorb- oder Bauchbereich der Person, beabstandet um einen zweiten Abstand (2; 3), ausgebildet sind.

3. Vorrichtung nach Anspruch 2, **dadurch gekennzeichnet, dass** die Auswerteeinheit zur Summen- oder Differenzbildung des ersten Abstandssignals mit einem vom zweiten Paar von Sensoreinheiten ausgegebenen zweiten Abstandssignal ausgebildet ist.

4. Vorrichtung nach Anspruch 1 bis 3, **dadurch gekennzeichnet, dass** die Auswerteeinheit zum zusätzlichen Erzeugen eines Pulsanzeigesignals ausgebildet ist, dass als Reaktion auf die erfassten, durch den menschlichen Herzschlag frequenz-, amplituden- und/oder signalformmäßig bestimmten Änderungen, insbesondere in einem Frequenzbereich zwischen 0,8 und 2,5 Hz, des ersten Abstandssignals erzeugt wird.

5. Vorrichtung nach einem der Ansprüche 1 bis 4, **gekennzeichnet durch** der Auswerteeinheit zugeordnete Analyse- und Speichermittel (26), die zum elektronischen Speichern des Atmungsanzeigesignals sowie zum Erfassen einer amplitu-den-, frequenz- und/oder signalformmäßigen Änderung des Atmungsanzeigesignals ausgebildet sind.

6. Vorrichtung nach Anspruch 5, **dadurch gekennzeichnet, dass** die Analyse- und Speichermittel zum Erzeugen eines Korrelationssignals zwischen dem Atmungsanzeigesignal und einem Pulsanzeigesignal ausgebildet sind.

7. Vorrichtung nach einem der Ansprüche 1 bis 6, **dadurch gekennzeichnet, dass** die Messeinrichtung und die Auswerteeinheit Bestandteil einer portablen, insbesondere batteriebetriebenen Einheit (10)sind, die bevorzugt mittels einer drahtlosen Datenverbindung (26) zur Übertragung des Anzeigesignals und/oder weiterer Signale mit einer stationären Basiseinheit verbindbar ist.

8. Vorrichtung nach einem der Ansprüche 1 bis 7, **dadurch gekennzeichnet, dass** die Auswerteeinheit zum zusätzlichen Erfassen einer durch Laufen oder Hüpfen der Person erzeugten Änderung des ersten Abstandssignals sowie zum Bestimmen einer Schritt- bzw. Hüpffrequenz daraus ausgebildet ist.

9. Vorrichtung nach einem der Ansprüche 1 bis 8, **dadurch gekennzeichnet, dass** die Auswerteeinheit zum Erfassen einer Hüllkurve des ersten Abstandssignals über einen gegenüber einer Atmungsfrequenz langen Zeitraum sowie zum Bestimmen einer Änderung eines unteren und/oder oberen Grenzwerts der Hüllkurve ausgebildet ist.

10. Vorrichtung nach einem der Ansprüche 1 bis 9, **dadurch gekennzeichnet, dass** die Sensoreinheiten als Ultraschallsensoren ausgebildet sind.

11. Verfahren zur Messdatenerzeugung betreffend atmungsbedingte Bewegungen der Bauchdecke und/oder des Brustkorbes einer Person zum Betreiben der Vorrichtung nach einem der Ansprüche 1 bis 10, **gekennzeichnet durch** die Schritte:
- kontinuierliches Messen eines ersten Abstandes zwischen zwei auf der Haut der Person an einem Brustkorboder Bauchbereich lösbar befestigten ersten Sensoren
- Auswerten von Abstandsänderungen des ersten Abstandes entsprechenden elektronischen Signaländerungen eines ersten Messsignals und
- Ausgeben eines Atmungsanzeigesignals als Reaktion auf eine periodische Signaländerung im Bereich zwischen 0,1 und 0,5 Hz.

12. Verfahren nach Anspruch 11, **gekennzeichnet durch** die Schritte:
- kontinuierliches Messen mindestens eines zweiten Abstandes zwischen zwei auf der Haut der Person an dem Brust- oder Bauchbereich lösbar befestigten zweiten Sensoren;
- Auswerten von Abstandsänderungen des zweiten Abstandes entsprechenden elektronischen Signaländerungen eines zweiten Messsignals und
- rechnerisches Verknüpfen des ersten und zweiten Messsignals zum Erzeugen eines Differenz- oder Summensignals daraus.

13. Verfahren nach Anspruch 11 oder 12, **gekennzeichnet durch** den Schritt:
- Erfassen von Abstandsänderungen des ersten Abstandes in einer **durch** Herzschlag bedingten Bewegung der Bauchdecke oder des Brustkorbes entsprechenden Periodendauer und Signalmuster sowie Ausgeben eines Pulsanzeigesignals als Reaktion darauf.

## Claims

1. Apparatus for producing measurement data concerning breathing-related movements of the abdominal wall and/or chest of a person, with
at least one first pair of sensor units (12, 14) which are designed for releasable attachment to the skin in a chest or abdomen region of the person, spaced apart by a first distance (1),
and a measuring device (20) which is connected to the first pair of sensor units and which is designed to detect electrically evaluatable signals of the sensor units as well as to generate a first distance signal corresponding to the first distance (1) and variations thereof,
wherein connected to the output of the measuring device is an evaluating unit (22, 28) which is designed to evaluate pulse-like and/or wave-like variations of the first distance signal in such a way that periodic signal variations can be detected, distinguished in frequency, amplitude or signal shape from periodic variations of the first distance signal determined by the human heartbeat and emitted as a breathing indicator signal which can be indicated electronically or further evaluated, **characterised in that** the signal variations have a frequency within the range between 0.5 and 0.1 Hz and the evaluating unit has means for detecting a signal shape of the variation of the first distance signal, wherein the signal shape detecting means are designed in such a way that the breathing indicator signal is emitted only as a reaction to a signal shape of the variation which is symmetrical in time lapse.

2. Apparatus according to claim 1, **characterised by** at least one second pair (16, 18) of sensor units connected to the measuring device, which are designed for releasable attachment to the skin in the chest or abdomen region of the person, spaced apart by a second distance (2; 3).

3. Apparatus according to claim 2, **characterised in that** the evaluating unit is designed for summation or subtraction of the first distance signal with a second distance signal emitted by the second pair of sensor units.

4. Apparatus according to claims 1 to 3, **characterised in that** the evaluating unit is designed to additionally generate a pulse indicator signal which is generated as a reaction to the detected variations of the first distance signal determined by the human heartbeat with respect to frequency, amplitude and/or signal shape, in particular within a frequency range between 0.8 and 2.5 Hz.

5. Apparatus according to any of claims 1 to 4, **characterised by** analysis and storage means (26) which are associated with the evaluating unit and which are designed for electronic storage of the breathing indicator signal and for detection of a variation of the breathing indicator signal in amplitude, frequency and/or signal shape.

6. Apparatus according to claim 5, **characterised in that** the analysis and storage means are designed to generate a correlation signal between the breathing indicator signal and a pulse indicator signal.

7. Apparatus according to any of claims 1 to 6, **characterised in that** the measuring device and the evaluating unit form part of a portable, in particular battery-operated unit (10) which preferably can be connected to a stationary base unit by means of a wireless data link (26) for transmission of the indicator signal and/or further signals.

8. Apparatus according to any of claims 1 to 7, **characterised in that** the evaluating unit is designed for additionally detecting a variation of the first distance signal produced by running or hopping of the person as well as for determining a step or hop frequency therefrom.

9. Apparatus according to any of claims 1 to 8, **characterised in that** the evaluating unit is designed to detect an envelope curve of the first distance signal over a long period compared with a breathing frequency as well as to determine a variation of a lower and/or upper limit value of the envelope curve.

10. Apparatus according to any of claims 1 to 9, **characterised in that** the sensor units are designed as ultrasound sensors.

11. Method for producing measurement data concerning breathing-related movements of the abdominal wall and/or chest of a person for operating the apparatus according to any of claims 1 to 10, **characterised by** the steps of:
- continuously measuring a first distance between two first sensors releasably attached to the skin of the person in a chest or abdomen region,
- evaluating electronic signal variations of a first measurement signal corresponding to distance variations of the first distance, and
- emitting a breathing indicator signal as a reaction to a periodic signal variation within the range between 0.1 and 0.5 Hz.

12. Method according to claim 11, **characterised by** the steps of:
- continuously measuring at least one second distance between two second sensors releasably attached to the skin of the person in the chest or abdomen region;
- evaluating electronic signal variations of a second measurement signal corresponding to distance variations of the second distance, and
- computer-linking the first and second measurement signals to generate a difference or sum signal therefrom.

13. Method according to claim 11 or 12, **characterised by** the step of:
- detecting period and signal pattern corresponding to distance variations of the first distance in a movement of the abdominal wall or chest caused by heart beating, and emitting a pulse indicator signal as a reaction thereto.

## Revendications

1. Dispositif pour produire des données de mesure concernant des mouvements d'origine respiratoire de la paroi abdominale et/ou de la cage thoracique d'une personne, comprenant :
- au moins une paire d'unités de capteur (12, 14) réalisée pour être fixée de façon désolidarisable sur la peau, sur une zone de cage thoracique ou de ventre de la personne, espacée de la valeur d'un premier espacement (1),
- et un organe de mesure (20) relié à la première paire des unités de capteur, réalisé pour détecter des signaux évaluables de façon électrique venant des unités de capteur, ainsi que pour générer un premier signal d'espacement qui correspond au premier espacement (1) ainsi qu'aux modifications de celui-ci,
dans lequel, en aval du dispositif de mesure, est raccordée une unité d'évaluation (22, 28), réalisée pour évaluer les modifications en forme d'impulsions et/ou en forme d'ondes du premier signal d'espacement, de sorte que des variations de signal périodiques sont détectées, sont, en termes de fréquence, d'amplitude, ou de forme de signal, distinguées des variations périodiques déterminées par le battement cardiaque humain, du premier signal d'espacement, et peuvent être fournies sous forme de signal respiratoire permettant un affichage et/ou une évaluation électronique supplémentaire, **caractérisé en ce que** les variations de signal présentent une fréquence dans la plage comprise entre 0,5 et 0,1 Hz et l'unité d'évaluation comporte des moyens pour détecter une forme de signal de la variation du premier signal d'espacement, les moyens de détection de forme de signal étant réalisés de manière que le signal indicateur de la respiration ne soit produit qu'en réaction à une forme de signal de la modification à déroulement temporel symétrique.

2. Dispositif selon la revendication 1, **caractérisé par** au moins une deuxième paire (16, 18) reliée au dispositif de mesure, d'unités de capteur, qui sont réalisées pour une fixation désolidarisable sur la peau, sur la zone de cage thoracique ou du ventre de la personne, espacées d'un deuxième espacement (2 ; 3).

3. Dispositif selon la revendication 2, **caractérisé en ce que** l'unité d'évaluation est réalisée pour la formation de la somme ou de la différence du premier signal d'espacement avec un deuxième signal d'espacement fourni par la deuxième paire d'unités de capteur.

4. Dispositif selon la revendication 1 à 3, **caractérisé en ce que** l'unité d'évaluation est réalisée pour produire, en plus, un signal indicateur d'impulsions, produit en réaction aux variations détectées du premier signal d'espacement, déterminées, en termes de fréquence, d'amplitude et/ou de forme de signal, par le battement cardiaque humain, en particulier dans une plage de fréquences comprise entre 0,8 et 2,5 Hz.

5. Dispositif selon l'une des revendications 1 à 4, **caractérisé par** des moyens d'analyse et de mémorisation (26), associés à l'unité d'évaluation et qui sont réalisés pour le stockage électronique du signal d'indication de respiration, ainsi que pour détecter une variation, concernant l'amplitude, la fréquence et/ou la forme de signal, du signal d'indication de respiration.

6. Dispositif selon la revendication 5, **caractérisé en ce que** les moyens d'analyse et de stockage sont réalisés pour produire un signal de corrélation, entre le signal d'indication de respiration et un signal d'indication d'impulsions.

7. Dispositif selon l'une des revendications 1 à 6, **caractérisé en ce que** l'organe de mesure et l'unité d'évaluation sont un composant d'une unité (10) portable, en particulier fonctionnement sur batterie, et qui, de préférence, est susceptible d'être reliée à une unité de base stationnaire au moyen d'une liaison de données (26) sans fil, pour transmettre le signal d'affichage et/ou d'autres signaux.

8. Dispositif selon l'une des revendications 1 à 7, **caractérisé en ce que** l'unité d'évaluation est réalisée pour détecter en plus une modification du premier signal d'espacement produite par une activité de course ou de sautillement de la personne, ainsi que pour déterminer à partir de cela une fréquence d'exécution des pas de course ou des sautillements.

9. Dispositif selon l'une des revendications 1 à 8, **caractérisé en ce que** l'unité d'évaluation est réalisée pour détecter une courbe enveloppe du premier signal d'espacement sur un intervalle de temps long par rapport à une fréquence respiratoire, ainsi que pour déterminer une variation d'une valeur limite inférieure et/ou supérieure de la courbe enveloppe.

10. Dispositif selon l'une des revendications 1 à 9, **caractérisé en ce que** les unités de capteur sont réalisées sous la forme de capteurs à ultrasons.

11. Procédé de génération de données de mesure concernant des mouvements d'origine respiratoire de la paroi abdominale et/ou de la cage thoracique d'une personne pour le fonctionnement du dispositif selon l'une des revendications 1 à 10, **caractérisé par** les étapes suivantes :
- mesure continue d'un premier espacement entre deux premiers capteurs, fixés de façon désolidarisable sur la peau de la personne, sur une zone de cage thoracique ou une zone de ventre,
- évaluation de variations de signal électronique d'un premier signal de mesure correspondant à des variations d'espacement du premier espacement, et
- fourniture d'un signal d'indication respiratoire, en réaction à une variation de signal périodique, dans la plage comprise entre 0,1 et 0,5 Hz.

12. Procédé selon la revendication 11, **caractérisé par** les étapes suivantes :
- mesure continue au moins d'un deuxième espacement entre deux deuxièmes capteurs, fixés de façon désolidarisable sur la peau de la personne dans une zone de poitrine ou de ventre,
- évaluation des variations de signaux électroniques, correspondant aux variations d'espacement du deuxième espacement, d'un deuxième signal de mesure, et
- combinaison par calcul, entre le premier et le deuxième signal de mesure, dans le but de produire à partir de cela un signal de différence ou de somme.

13. Procédé selon la revendication 11 ou 12, **caractérisé par** l'étape suivante :
- détection de la durée de période et du modèle de signal, correspondant aux variations d'espacement du premier espacement dans un déplacement conditionné par le battement cardiaque, de la paroi abdominale ou de la cage thoracique, ainsi que fourniture d'un signal d'affichage d'impulsions en réaction à cela.
